# EUROPÄISCHE PATENTSCHRIFT

(11) **EP 0 496 362 B1**
(45) Veröffentlichungstag und Bekanntmachung des Hinweises auf die Patenterteilung: **18.10.1995**
(21) Anmeldenummer: 92100948.6
(22) Anmeldetag: 21.01.1992
(51) Int. Cl.: C07C 51/54, C07D 319/06, C07C 55/08

(54) **Verfahren zur Herstellung von Malonsäurederivaten**
Process for preparing malonic anhydride
Procédé de préparation d'anhydride malonique

(30) Priorität: 21.01.1991 CH 156/91; 21.01.1991 CH 157/91
(43) Veröffentlichungstag der Anmeldung: 29.07.1992
(60) Teilanmeldung: 94114552.6
(73) Patentinhaber: LONZA AG, CH-3945 Gampel/Wallis (CH)
(72) Erfinder: O'Murchu, Colm, Dr., Visp (Kanton Wallis) (CH)
(74) Vertreter: Weinhold, Peter, Dr.

(56) Entgegenhaltungen:
- US-A- 4 360 691
- BULLETIN DES SOCIETES CHIMIQUES BELGES, Bd.86, Nr.12, 1977, BRUSSELS Seiten 991 - 1002 P CROOY ET AL
- CHEMICAL ABSTRACTS, vol. 84, no. 13, 29. März 1976, Columbus, Ohio, US; abstract no. 90094v, A KOST ET AL 'Meldrums acid and its analogs' Seite 524 ;Spalte 1 ;

## Beschreibung

Malonsäureanhydrid ist ein reaktiver Baustein zur Herstellung zahlreicher Wirkstoffe für Pharmazeutika, Pestizide oder Farbstoffe (Lit. US-PS 4 251 447, US-PS 4 360 691).

Ozonisierungen von Diketen zum Malonsäureanhydrid sind bekannt.

So beansprucht Perrin in der US-PS 4 251 447 und der US-PS 4 360 691 die Herstellung von Malonsäureanhydrid bzw. von Malonsäureanhydridderivaten durch Umsetzung von Diketen bei 0°C bis -100°C mit Ozon. Ein aus Sicherheitsgründen Schwerwiegender Nachteil dieses Verfahrens ist aber, dass als Nebenprodukt in organischen Lösungsmitteln unlösliche Formaldehydperoxid-Polymere entstehen (US-PS 4 360 691, Spalte 5, Zeile 3-5), die z.B. gemäss Lapalme et al., Can.J.Chem. 57, S.3272 (1979) hochexplosiv sind.

Die Aufgabe bestand folglich darin, ein Verfahren zu entwickeln, das sicherheitstechnisch unbedenklich ist und im technischen Massstab realisierbar ist.

Das grosstechnisch verfügbare Diketen wird nach dem erfindungsgemäßen Verfahren zunächst bei einer Temperatur zwischen -30°C und -100°C in Gegenwart einer Carbonylverbindung der allgemeinen Formel 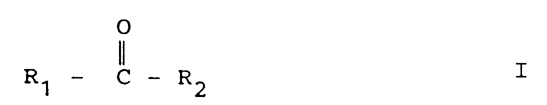
worin R₁ und R₂ gleich oder verschieden sind und Wasserstoff, eine Alkylgruppe, eine Alkoxygruppe oder eine Arylgruppe bedeuten, zum Malonsäureanhydrid der Formel 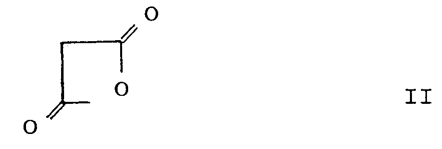
ozonisiert.

Da sich das Malonsäureanhydrid bei Temperaturen von -30°C und höher in Keten und CO₂ zersetzt, wird es in der Regel direkt weiterverarbeitet, z.B. gemäss US-PS 4 251 447, z.B. durch Umsetzung mit Alkoholen, Phenolen oder Aminen in die entsprechenden Malonsäurederivate. Daneben kann Malonsäureanhydrid durch Reaktion mit Ketonen, insbesondere mit Aceton, zu den entsprechenden 2,2-disubstituierten 4,6-Dioxo-1,3-dioxanen umgesetzt werden (vergl. EP-A-94 114 552.6).

Das Malonsäureanhydrid muß jedoch nicht unbedingt weiter umgesetzt werden, sondern kann bei Temperaturen unter -30°C, insbesondere bei Temperaturen unter -40°C, konserviert werden.

Die Ozonisierung in Gegenwart einer der genannten Carbonylverbindungen bewirkt, dass anstelle der Bildung der Formaldehydperoxid-Polymere lösliche Ozonide gebildet werden, die gefahrlos abgetrennt werden können. Dies geschieht zweckmäßig nach erfolgter Weiterumsetzung bei der Aufarbeitung der Malonsäurederivate. Im Falle der Umsetzung mit Ketonen erfolgt die Aufarbeitung und Trennung vorteilhaft mittels Chromatographie der Produkte.

Bei den Resten R₁ und R₂ bedeutet Alkyl zweckmässig eine Nieder alkylgruppe mit 1-4 C-Atomen, vorzugsweise eine Methyl-, Ethyl-, Propyl-, n-Butyl-, Isobutyl- oder tert-Butylgruppe. Alkoxy bedeutet zweckmässig eine Methoxy- oder Ethoxygruppe. Aryl bedeutet zweckmässig Phenyl oder p-Tolyl, vorzugsweise Phenyl.
Zweckmässig werden als Carbonylverbindungen Aldehyde der allgemeinen Formel 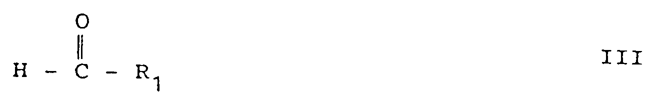
worin R₁ die genannte Bedeutung hat, eingesetzt.

Bevorzugte Aldehyde sind Formaldehyd, Acetaldehyd, Propionaldehyd, Isobutyraldehyd, Pivalaldehyd, Benzaldehyd oder p-Tolylaldehyd. Besonders bevorzugt wird Acetaldehyd eingesetzt.

Zweckmässig werden die Carbonylverbindungen stöchiometrisch oder im leichten Überschuss, bezogen auf Diketen, eingesetzt.

Die Reaktionstemperatur bewegt sich zwischen -30°C und -100°C. Da, wie eingangs erwähnt, sich das Malonsäureanhydrid bei -30°C zu zersetzen beginnt, wird vorzugsweise bei einer Temperatur unter -30°C, vorzugsweise zwischen -40°C und -80°C, gearbeitet

Von Vorteil erfolgt die Reaktion in Gegenwart von aprotischen, wasserfreien Lösungsmitteln, wie z.B.
in Alkanen, z.B. Pentan, Hexan;
in aromatischen Kohlenwasserstoffen, z.B. C₁-C₄-alkylsubstituierten Benzolen, wie z.B. Toluol, Ethylbenzol, Dimethylbenzol, Ethylmethylbenzol, Propylbenzol, Isopropylbenzol, Butylbenzol, Isobutylbenzol, Diethylbenzol, Mesitylen, Cymol; halogenierten Benzolen, wie z.B. Fluorbenzol, Chlorbenzol; in Ethern, z.B. Dimethylether, Diethylether, Dipropylether, Dibutylether, tertiär-Butylmethylether, Tetrahydrofuran, Dioxan; in Estern, z.B. C₁-C₈-Alkylformiaten, wie Methylformiat, Ethylformiat, Propylformiat, Isobutylformiat, Hexylformiat, Octylformiat; C₁-C₈-Alkylacetaten, wie Methylacetat, Ethylacetat, Propylacetat, Butylacetat, 2-Ethylhexylacetat, Pentylacetat, Hexylacetat, Heptylacetat, Octylacetat;
in Nitrilen, z.B. Acetonitril, Propionitril, Butyronitril oder in halogenierten Kohlenwasserstoffen, z.B. Methylenchlorid, Chloroform.

In der Regel wird so vorgegangen, dass man Ozon, welches als Gasgemisch mit Hauptanteil Sauerstoff generiert wird, in eine Lösung von Diketen und entsprechender Carbonylverbindung in einem der genannten Lösungsmittel so lange einleitet, bis Blaufärbung der Lösung das Ende der Ozonisierung anzeigt oder bis die Stöchiometrische Menge Ozon absorbiert ist.

Die folgenden Beispiele illustrieren das erfindungsgemäße Verfahren zur Herstellung von Malonsäureanhydrid.

### Verfahren zur Herstellung von Malonsäureanhydrid

### Beispiel 1:

Eine Lösung von 4,2 g (0,050 Mol) Diketen und 2,2 g (0,050 Mol) Acetaldehyd in 100 ml Chloroform wurde bei -60°C mit einem Strom 4%igen Ozons in Sauerstoff bis zur Blaufärbung der Lösung ozonisiert.
Das überschüssige Ozon wurde zuerst mit Sauerstoff, dann mit Stickstoff gespült. Charakterisierung des Reaktionsproduktes: ¹H-NMR: (Reaktionsgemisch bei -50°C, 300 MHz) δ in ppm:

| | |
|---|---|
| Malonsäureanhydrid: | 4,24, s |
| Propylenozonid: | 1,52, d, CH₃, |
| | 5,1, s, CH, |
| | 5,31, q, CH, |
| | 5,32, s, CH. |

### Beispiel 2:

Eine Lösung von 4,2 g (0,050 Mol) Diketen und 4,0 g (0,055 Mol) Isobutyraldehyd in 200 ml Chloroform wurde bei -60°C mit einem Strom 4%-igen Ozons in Sauerstoff bis zur Blaufärbung der Lösung ozonisiert. Das überschüssige Ozon wurde mit Stickstoff gespült.
¹H-NMR: (Reaktionsgemisch bei -50°C, 300 MHz) δ in ppm:

| | |
|---|---|
| Malonsäureanhydrid: | 4,24, s |
| 3-Isopropyl-1,2,4-trioxolan: | 1,03, dd, CH₃, |
| | 2,00, m, CH, |
| | 4,93, d, CH, |
| | 5,07, s, CH, |
| | 5,31, s, CH. |

### Beispiel 3:

Eine Lösung von 4,2 g (0,050 Mol) Diketen und 4,3 g (0,055 Mol) Pivalaldehyd in 200 ml Chloroform wurde bei -60°C mit einem Strom 4%-igen Ozons in Sauerstoff bis zur Blaufärbung der Lösung ozonisiert. Das überschüssige Ozon wurde mit Stickstoff gespült.
¹H-NMR: (Reaktionsgemisch bei -50°C, 300 MHz) δ in ppm:

| | |
|---|---|
| Malonsäureanhydrid: | 4,24, s |
| 3-Tertiärbutyl-1,2,4-trioxolan: | 1,01, s, CH₃, |
| | 4,82, s, CH, |
| | 5,01, s, CH, |
| | 5,35, s, CH. |

### Beispiel 4:

Eine Lösung von 4,2 g (0,050 Mol) Diketen und 5,84 g (0,055 Mol) Benzaldehyd in 200 ml Chloroform wurde bei -60°C mit einem Strom 4%-igen Ozons in Sauerstoff bis zur Blaufärbung der Lösung ozonisiert. Das überschüssige Ozon wurde mit Stickstoff gespült.
¹H-NMR: (Reaktionsgemisch bei -50°C, 300 MHz) δ in ppm:

| | |
|---|---|
| Malonsäureanhydrid: | 4,23, s |
| 3-Phenyl-1,2,4-trioxolan | 5,41, s, CH, |
| | 5,57, s, CH, |
| | 6,07, s, CH, |
| | 7,4-7,8, m, Ph. |

### Beispiel 5:

Eine Lösung von 4,2 g (0,050 Mol) Diketen und 6,0 g (0,055 Mol) p-Toluylaldehyd in 200 ml Chloroform wurde bei -60°C mit einem Strom 4%-igen Ozons in Sauerstoff bis zur Blaufärbung der Lösung ozonisiert. Das überschüssige Ozon wurde mit Stickstoff gespült.
¹H-NMR: (Reaktionsgemisch bei -50°C, 300 MHz) δ in ppm:

| | |
|---|---|
| Malonsäureanhydrid: | 4,22, s |
| 3-(4-Methylphenyl)-1,2,4-trioxolan | 2,42, s, CH₃, |
| | 5,39, s, CH, |
| | 5,56, s, CH, |
| | 6,03, s, CH, |
| | 7,46, d, aromatische CH. |

### Beispiel 6:

In derselben Weise, wie in Beispiel 1 angegeben, aber mit 100 ml Dichlormethan als Lösungsmittel, wurde eine Lösung von Malonsäureanhydrid und Propylenozonid erhalten.

### Beispiel 7:

In derselben Weise, wie in Beispiel 1 angegeben, aber mit 100 ml Toluol als Lösungsmittel, wurde eine Lösung von Malonsäurenahydrid und Propylenozonid erhalten.

## Patentansprüche

1. Verfahren zur Herstellung von Malonsäurederivaten durch Ozonisierung von Diketen, dadurch gekennzeichnet, daß Diketen bei einer Temperatur zwischen -30°C und -100°C in Gegenwart einer Carbonylverbindung der allgemeinen Formel 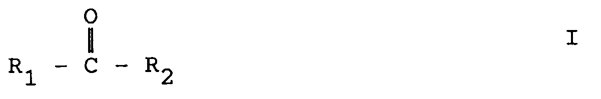 worin R₁ und R₂ gleich oder verschieden sind und Wasserstoff, eine Alkylgruppe, eine Alkoxygruppe oder eine Arylgruppe bedeuten, zum Malonsäureanhydrid der Formel 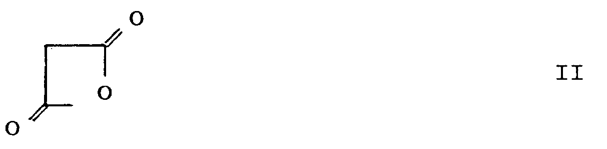 ozonisiert wird und dieses gegebenenfalls mit Alkoholen, Aminen oder Ketonen weiter zu den entsprechenden Malonsäurederivaten umgesetzt wird.

2. Verfahren nach Patentanspruch 1, dadurch gekennzeichnet, daß das Malonsäureanhydrid bei Temperaturen unter -30°C, inbesondere bei Temperaturen unter -40°C konserviert wird.

3. Verfahren nach mindestens einem der Patentansprüche 1 oder 2, dadurch gekennzeichnet, dass als Carbonylverbindung ein Aldehyd der Formel 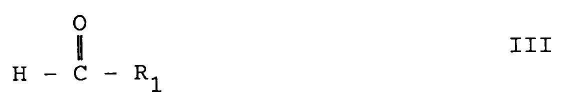 worin R₁ die genannte Bedeutung hat, eingesetzt wird.

4. Verfahren nach mindestens einem der Patentansprüche 1, 2 oder 3, dadurch gekennzeichnet, dass als Carbonylverbindung Acetaldehyd eingesetzt wird.

5. Verfahren nach mindestens einem der Patentansprüche 1 bis 4, dadurch gekennzeichnet, dass die Ozonisierung bei einer Temperatur zwischen -40°C und -80°C, in Gegenwart eines aprotischen, wasserfreien Lösungsmittels erfolgt.

## Claims

1. Process for the preparation of malonic acid derivatives by ozonization of diketene, characterized in that diketene is ozonized at a temperature of from -30 °C to -100 °C in the presence of a carbonyl compound of general formula 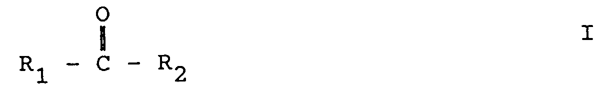 wherein R₁ and R₂ are the same or different and represent hydrogen, an alkyl group, an alkoxy group or an aryl group, to give malonic anhydride of formula 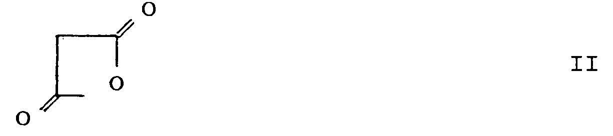 which is optionally further reacted with alcohols, amines or ketones to the respective malonic acid derivatives.

2. Process according to claim 1, characterized in that the malonic anhydride is stored at a temperature below -30 °C, particularly at temperatures below -40 °C.

3. Process according to at least one of claims 1 or 2, characterized in that an aldehyde of formula 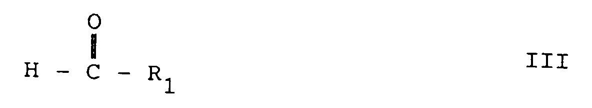 wherein R₁ has the mentioned meaning, is used as the carbonyl compound.

4. Process according to at least one of claims 1, 2 or 3, characterized in that acetic aldehyde is used as the carbonyl compound.

5. Process according to at least one of claims 1 to 4, characterized in that the ozonization is carried out in the presence of an aprotic, anhydrous solvent at a temperature between -40 °C to -80 °C.

## Revendications

1. Procédé pour la préparation de dérivés de l'acide malonique par ozonisation de dicétène, caractérisé en ce que le dicétène est ozonisé à inne température entre -30°C et -100°C en présence d'un composé carbonyle de la formule générale 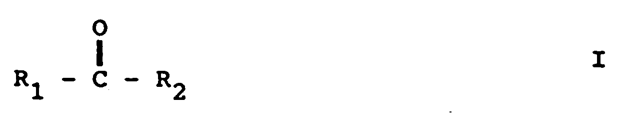 dans laquelle R₁ et R₂ sont identiques ou différents et signifient hydrogène, un groupe alkyle, un groupe alcoxy ou un groupe aryle, pour obtenir un anhydride de l'acide malonique de la formule 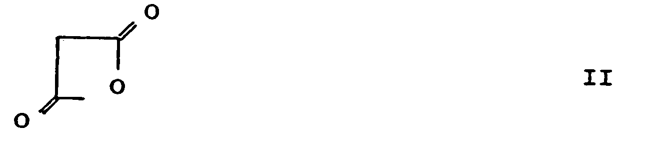 et celui-ci est éventuellement mis en réaction avec des alcools, des amines ou des cétones pour donner les dérivés correspondants de l'acide malonique.

2. Procédé selon la revendication 1, caractérisé en ce que l'anhydride de l'acide malonique est conservé à des températures inférieures à -30°C, en particulier à des températures inférieures à -40°C.

3. Procédé selon au moins l'une des revendications 1 ou 2, caractérisé en ce qu'en tant que composé carbonyle, on met en oeuvre un aldéhyde de la formule 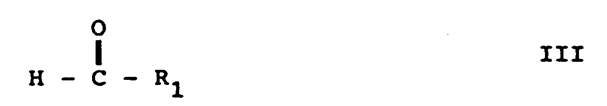 dans laquelle R₁ a la signification citée.

4. Procédé selon au moins l'une des revendications 1, 2 ou 3, caractérisé en ce qu'en tant que composé carbonyle, on met en oeuvre de l'acétaldéhyde.

5. Procédé selon au moins l'une des revendications 1 à 4, caractérisé en ce que l'ozonisation s'effectue à une température entre -40°C et -80°C en présence d'un solvant anhydre aprotique.
